## Europäisches Patentamt
## European Patent Office
## Office européen des brevets

(19)

(11) Publication number: **0 054 521**
**A1**

(12)

# EUROPEAN PATENT APPLICATION

(21) Application number: **81830230.9**

(22) Date of filing: **19.11.81**

(51) Int. Cl.³: **A 61 M 17/00**

(30) Priority: **04.12.80 IT 2644780**

(43) Date of publication of application:
**23.06.82 Bulletin 82/25**

(84) Designated Contracting States:
**AT CH DE FR GB LI LU NL SE**

(71) Applicant: **X LABORATIES ITALIA S.p.A:**
**2 Via Agnello**
**I-20122 Milan(IT)**

(72) Inventor: **Benetti, Alfredo**
**8, Via Cà di Cozzi**
**Verona(IT)**

(72) Inventor: **Barbo, Italo**
**50, Via Ivrea**
**Montalto Dora(IT)**

(74) Representative: **Perani, Aurelio et al,**
**c/o JACOBACCI-CASETTA & PERANI S.N.C. Via Visconti di Modrone 7**
**I-20122 Milano(IT)**

(54) Gas evacuator, particularly for anaesthetic inhalation apparatus.

(57) A gas evacuator (1) for anaesthetic gases exhausted from an anaesthetic inhalation apparatus comprises a variable-volume chamber (11) for the continuous reception of the gases, and an aspirator-pusher unit (17, 18) for the continuous evacuation of the gases from the chamber (11). The aspirator-pusher unit (17, 18) is structurally independent of the variable-volume chamber (11), and is driven by a motor (27) the speed of which is controlled and adjusted as a function of the pressure of the gases in the chamber (11). The evacuator can be connected directly to the mouth of the patient being anaesthetised.

FIG. 1

EP 0 054 521 A1

Gas evacuator, particularly for anaesthetic inhalation apparatus

The present invention relates to a gas evacuator, particularly for gases exhausted from anaesthetic inhalation apparatus.

Anaesthetic inhalation apparatus are always equipped with one or more suitable valve units which ensure exhaustion both of the anaesthetic gases exhaled by the patient and any gas which may be delivered in excess of the quota used by the patient. This exhaustion normally occurs into the same environment as that in which the anaesthesis takes place, and the subsequent pollution may reach levels which are perhaps dangerous and, in any case, unacceptable.

When an anaesthetic inhalation apparatus is functioning, therefore, it is necessary to provide for the evacuation of the gases exhausted from the apparatus. In order to achieve this, various pumping systems (aspirator-pusher units) have been proposed and tested, which are connected directly to the exhaust valve units of the anaesthetic apparatus to convey the anaesthetic gases away from the environment. Use of these pumping systems has revealed a technical problem consisting essentially of the fact that, in the region of the exhaust units, there must be no obstacles to the outflow of the gases nor any aspiration effects on the gases, otherwise the patient's breathing could be seriously compromised and, in any case, there would be interference with the normal course of anaesthesia. In fact, the patient must always be assured of both effortless exhalation and inhalation of the quantity of gas necessary for anaesthesia.

It has been amply demonstrated that the pumping systems provided and used until now do not have the structural and functional characteristics to resolve the aforesaid problem satisfactorily, and, furthermore, they have proved complex, difficult and costly to produce, as well as extremely cumbersome.

The idea for solving the aforesaid technical problem, which is the basis for this invention, is to have a variable-volume chamber which is always able to receive the gases exhausted from the anaesthetic apparatus and in which the consequent variations of volume will occur without having to overcome any substantial resistance, and an aspirator-pusher unit which can continuously evacuate the gases as soon as they are received in the chamber.

Moreover, by correlating the intake capacity of the aspirator-pusher unit with the existing volume of the variable-volume chamber, it is possible to maintain the pressure of the gas in the chamber within a predetermined and limited range of values. This range of values for the pressure is determined, imposed, and controlled in such a way as to prevent any resistance to the outflow of the anaesthetic gases, as well as any aspiration effects thereon, in the region of the exhaust valve of the anaesthetic apparatus.

According to the present invention, therefore, there is provided a gas evacuator, particularly for anaesthetic inhalation systems, characterised in that it comprises:

- a variable-volume chamber in fluid communication with a gas inlet pipe;

- an aspirator-pusher unit having an aspiration inlet communicating with the variable-volume chamber and a force-discharge outlet communicating with a gas delivery-exhaust duct;

- a by-pass pipe, with respective valve members, between the aspiration inlet and the force-discharge outlet of the aspirator-pusher unit for by-passing said unit,

- a variable-speed electric motor for driving the aspirator-pusher unit,

- a volume sensor for continuously registering the volume of the chamber and controlling speed variations, of the electric motor as a function of the volume, and

- means for operating the valve members of the by-pass pipe.

In a preferred embodiment of the invention, the variable-volume chamber comprises a cylindrical bellows having a fixed rigid end and a movable rigid end, and the volume sensor comprises a position transducer which continuously registers the distance between the movable rigid end and the fixed rigid end.

The main advantage of the evacuator of this invention lies in the fact that the aspirator-pusher unit is able to draw air from the variable-volume chamber in an automatically controlled quantity so that it does not flow into the gas inlet tube at pressures greater than about 5 mm of water, while the gases drawn from the variable-volume chamber can be evacuated at pressures up to a maximum of 300 cm of water.

Other advantages include the simplicity of construction and operation, the small size and, above all, safety and consistently reliable operation.

Further characteristics and advantages will become apparent from the following detailed description of one embodiment of an evacuator according to the invention, made with reference to the accompanying drawings supplied purely by way of illustrative and non-limitative example.

In the drawings:

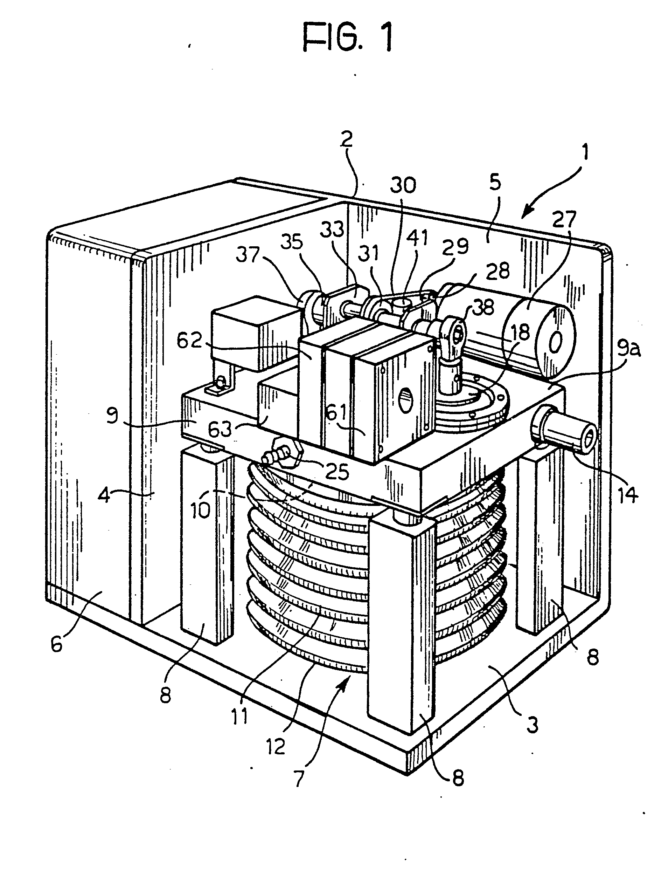

Figure 1 is a perspective view of an evacuator according to the invention;

Figure 2 is a cross-section on an enlarged scale of a detail of the evacuator of Figure 1;

Figure 3 is a partial section of a further constructional detail of the evacuator in Figure 1, and

Figures 4 and 5 show in section and plan view, respectively, a safety valve for the evacuator of this invention.

With reference to the drawings, a gas evacuator 1 according to the invention includes an essentially box-shaped support structure 2 which defines a base plate 3, and a pair of walls 4, 5, preferably covered with sound-proofing material, which are disposed at an angle and, with the base plate 3, form a pair of adjacent spaces 6, 7.

In the space 7, four uprights 8 support a rectangular plate 9 of a predetermined suitable thickness, which is parallel to the base plate 3. A circular collar 10 of predetermined height and diameter is formed on the face of the plate 9 facing the base plate 3. To this collar 10 there is fitted and fixed, by known means, the upper opening of a bellows 11 of very soft rubber, which has a rigid bottom end 12. The bellows 11 constitute a cylindrical variable-volume chamber which is connectible to the exhaust valve (not shown) of an anaesthetic inhalation apparatus (also not shown) for collecting the exhausted anaesthetic gases. For this purpose, a gas inlet pipe 13 is formed in the thickness of the plate 9, which has one end opening into the bellows 11 and the other end opening on one side of the plate 9 and provided with a tubular pipe union 14 for the gas.

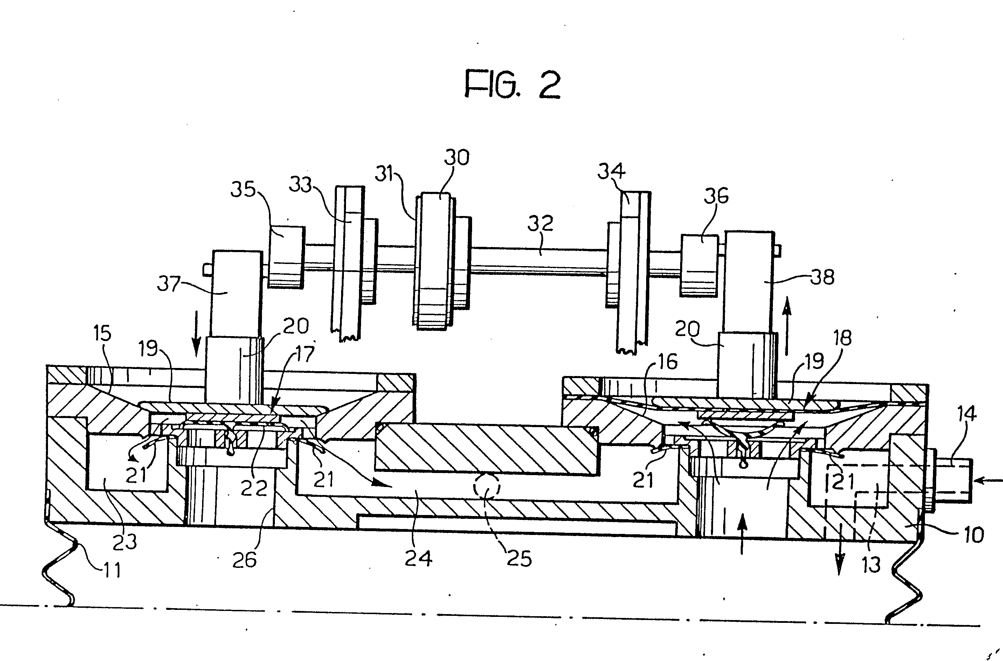

On the upper face 9a of the plate 9 are two identical circular seatings 15, 16 in which respective identical diaphragm pumps 17, 18 of conventional type are positioned. The pumps 17, 18 are shown in cross-section in Figure 2, and these sections show the reinforcing washers 19 of the diaphragms, to which there is fixed axially a rod 20 for operating the diaphragm pump, as will be better seen in the later description. As shown diagrammatically, a non-return flap valve 21 is located over the delivery of the pump 17 (18), while a non-return flap valve 22 is located over the intake of the pump 17. More particularly, the delivery of the pump 17 opens into an annular chamber 23, which is made in the plate 9 coaxially with the seating 15 of the pump, and is in fluid communication with an exhaust duct 25 common to both pumps 17, 18 through a passage 24. The intake of the pump 17 (18) opens into a duct which, in turn, opens into the bellows 11.

The diaphragm pumps 17, 18 are operated by an electric motor 27 which is supported by the plate 9 and has a pulley 29 keyed onto its drive shaft 28. A positive drive belt 30 is fitted onto the pulley 29 and a pulley 31 keyed on to a shaft 32 which is supported by a pair of bearings 33, 34 carried by the plate 9. At opposite ends of the shaft 32 are mounted respective eccentrics 35, 36 (cranks) with which the upper ends of connecting rods 37, 38 are coupled in the usual manner. The lower ends of these connecting rods are fixed to the rods 20 of the reinforcing washers 19 of each diaphragm pump 17, 18. The eccentrics 35, 36 are mutually staggered so that, when one diaphragm pump is on its intake stroke, the other diaphragm pump is on its delivery stroke.

The speed of the electric motor 27 is controlled by a conventional electronic circuit, not shown, and is correlated proportionally to the variable volume of the bellows 11. More particularly, the speed of the electric motor 27 increases as the volume of the bellows increases, and decreases as the latter decreases.

For this purpose, a preferred, but not restrictive, embodiment employs a position transducer 39 which consists essentially of a rod 40 movable axially in a coil 41 which is formed by primary and secondary windings 42, 43, and is fixedly supported by the plate 9. The free, lower, end of the rod 40 registers the distance of the rigid bottom end 12 of the bellows 11 from the face of the plate 9 and, upon variation of this distance, the length of the rod within the coil 41 varies.

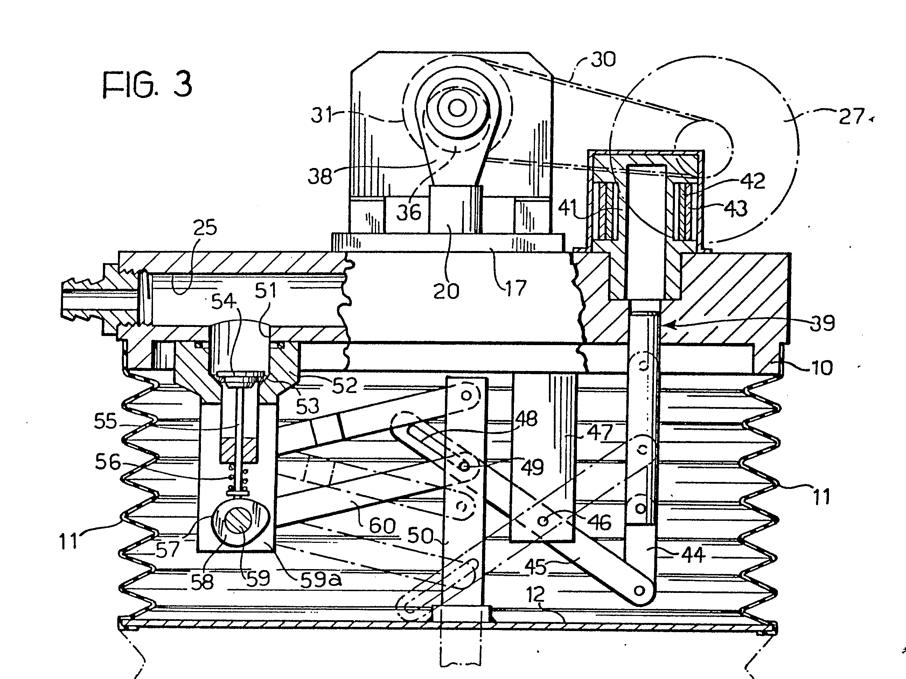

With particular reference to Figure 3, the lower free end of the rod 40 is pivoted to one end of a link 44, the other end of which is pivoted to a lever 45. This lever 45 is articulated, in an intermediate position 46, to a rod-shaped support 47 which is fixed to the plate 9 and extends into the bellows 11 parallel to the direction of movement of the rigid end 12 of the latter. Adjacent its other end, the lever 45 has a longitudinal slot 48 engaged by a pin 49 which is carried by a rod-shaped support 50 fixed to the centre of the rigid end 12 of the bellows and extending axially therefrom. The mechanical linkage between the rod 40 of the position transducer 39 and the rigid end 12 of the bellows is such that, when the rigid end 12 moves away from the plate 9, the rod 40 moves further into the coil 41. As the rigid end 12 of the bellows 11 approaches the plate 9, the rod 40 of the transducer assumes positions progressively further outside the coil 41. The variations of the position of the rod 40 in the coil 41 determine variations in the magnetic field and, consequently, variations in the current of the secondary winding 43 of the coil. These current variations act on the electronic motor-speed control circuit in a predetermined and desired manner.

The exhaust duct 25 communicates with the interior of the bellows 11 through an opening 51 made through the thickness of the plate 9 and a tubular union 52 fixed to the plate in correspondence with the opening. The union 52 defines a valve seating 53 with which an essentially disc-type poppet valve 54 cooperates. The stem 55 of the poppet valve is subject to the action of a spring 56 which urges the valve 54 into its closed position in the valve seating 53. The lower, free, end of the valve stem 55 bears upon the profile 57 of a cam 58 keyed onto a pin 59 which is rotatably supported by supports 59a fixed to the plate 9 and extending into the bellows 11 parallel to the direction of movement of its rigid bottom end 12. The pin 59 is keyed on to the end of a connecting rod 60, the other end of which is fixed for rotation on the pin 49 of the support 50 mentioned previously.

The position of the pin 59 in the bellows 11 and the shape of the cam 58 are predetermined so that, when the rigid bottom end 12 of the bellows has reached a minimum predetermined distance from the plate 9, the cam 58 has raised the stem 55 against the action of the spring 56, whereby the valve 54 is moved off the valve seating 53. In this condition, the exhaust duct 25, and hence the deliveries of the diaphragm pumps 17, 18, are in communication with the interior of the bellows 11. Consequently, the diaphragm pumps 17, 18 will be "bypassed" in the sense that the fluid drawn through the bellows 11, by the diaphragm pumps, is diverted back into the bellows through the open valve seating 53.

The opening 51, the union 52 and the valve seating 53 in its open condition, in practice, constitute a by-pass which connects the deliveries of the diaphragm pumps with the intakes, by-passing the pumps.

It should be pointed out that, in the absence of the by-pass duct and the control means (55, 56) for opening the respective valve member (54, 53), a low pressure would be created in the bellows 11 when the electric motor 27 reaches its minimum speed of rotation, which would be harmful to the patient undergoing anaesthesia.

Consequently, the minimum distance from the movable rigid end 12 of the bellows to the plate 9 (which, in substance, constitutes the fixed rigid end of the bellows), and hence the minimum volume of the bellows, are predetermined in strict correspondence with the minimum rotational speed of the motor 27.

Advantageously, the evacuator 1 is equipped with a conventional pressure transducer, not shown, which is capable of registering pressure variations in the bellows 11 of the order of millimetres of water, and transmitting these values to a conventional visual display, also not shown. There is provision for the use of a comparator system and electronic amplification for transmitting the data registered by the pressure transducer to the visual display.

The evacuator 1 is further provided with safety valves 61, 62 located in the intake, that is, the gas inlet pipe 14, and a valve 63 in the delivery, that is, the gas exhaust duct 25.

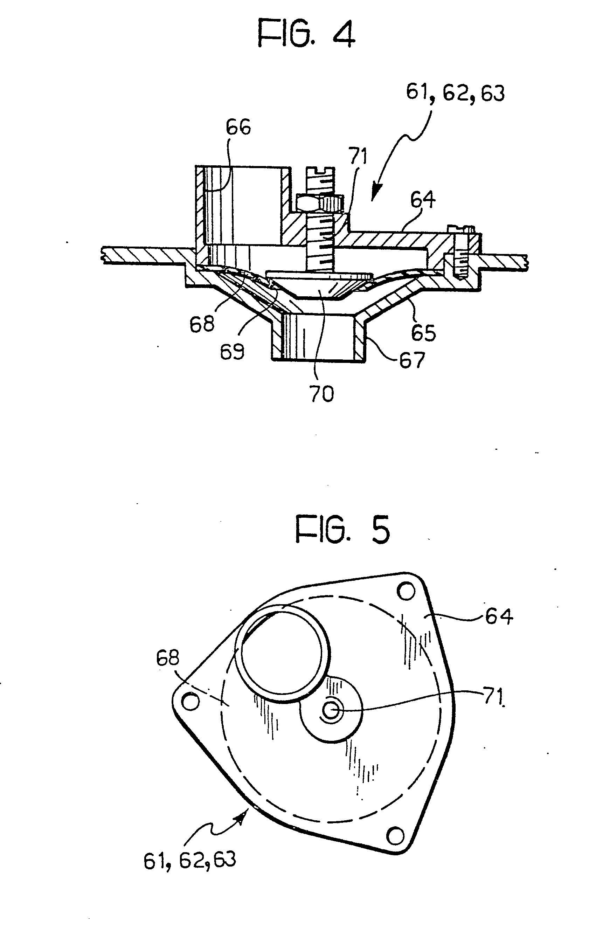

The valves 61, 62, 63 are structurally identical and are of the type shown diagrammatically in Figure 4.

The valve body comprises two juxtaposed elements 64, 65 which are fixed together by conventional means, such as screws placed at $120^{\circ}$ from one another. The elements 64, 65 define respective ducts 66, 67 for the passage of gas, and retain between them a circular diaphragm 69 with a circular central hole 69. Cooperating with this circular hole is the conical head 70 of a stem 71 carried axially, in an adjustable manner, by the element 64. The gas acting upon the diaphragm 68 lifts the latter from the conical head 70, and the gas is exhausted to the environment at the calibration pressure.

The valves 61, 62 are for high and low pressure respectively, and are adjustable in a range from 0 to 50 mm of water. The valve 63 is a high pressure valve which is adjustable between 200 and 300 cm of water, and intervenes in the case of an accidental obstruction of the exhaust tubing of the evacuator of this invention.

Advantageously, the intervention or automatic operation of the valves 61, 62, 63 is monitored and signalled acoustically or by a light signal.

It is pointed out that, in addition to the advantages mentioned above, the evacuator of this invention has the further advantage of being connectible directly to the mouth of a patient for safety of operation and for the characteristics offered thereby, that is, practically no resistance, nor any aspiration, when the patient exhales.

0054521

- 1 -

CLAIMS

1. Gas evacuator particularly for anaesthetic inhalation apparatus, characterised in that it comprises:
- a variable-volume chamber (11) in fluid communication with a gas inlet pipe (13,14);
- an aspirator-pusher unit (17,18) having an aspiration inlet (22) communicating with the variable-volume chamber (11), and a force-discharge outlet (21) communicating with a gas delivery-exhaust duct (25);
- a by-pass pipe (51,52) with respective valve members (53, 54, 55) between the aspiration inlet (22) and the force-discharge outlet (21) of the aspirator-pusher unit (15,18) for by-passing said unit;
- a variable speed electric motor (27) for driving the aspirator-pusher unit (17,18);
- a volume sensor (39) for continuously registering the volume of the chamber (11) and controlling variations in speed of the electric motor (27) as a function of the volume, and
- means for operating (59, 58, 57, 56) the valve members (55, 54, 53) of the by-pass pipe (51,52).

2. Evacuator as claimed in Claim 1, in which the variable-volume chamber comprises a cylindrical bellows (11) having a fixed rigid end (9) and a movable rigid end (12), and in which the volume sensor (39) comprises a position transducer (40,41) which continuously registers the distance between the movable rigid end (12) and the fixed rigid end (9).

3. Evacuator as claimed in Claim 2, in which the position transducer comprises a coil and a rod (40) movable axially in the coil, the rod (40) having one end connected to the movable rigid end (12) of the bellows (11).

4. Evacuator as claimed in any of the preceding claims, in which it further comprises two safety valves (61,62) located in the gas inlet pipe (14), and one safety valve (63) located in the gas delivery-exhaust duct (25).

5. Evacuator as claimed in Claim 4, in which each safety valve comprises an identical valve body including two juxtaposed elements (64,65) which are fixed together and retain between them a circular diaphragm (68), the diaphragm having a central hole (69) with which cooperates the conical head (70) of a valve stem (71) supported axially and adjustably by one of said elements (64).

6. A method of evacuating gases exhausted from an anaesthetic inhalation apparatus, characterised in that:
- the gases are received continuously in a variable-volume chamber (11) which offers no substantial resistances to the volume variations, and
- the gases are evacuated continuously from the chamber by an aspirator-pusher unit (17,18) which is structurally independent of the chamber (11), the pressure of the gases in the chamber being maintained within a predetermined range of values.

# FIG. 1

# FIG. 2

FIG. 3

## FIG. 4

66    61, 62, 63    71    64    68    69    65    67    70

## FIG. 5

68    64    71    61, 62, 63

**European Patent Office**

## EUROPEAN SEARCH REPORT

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| A | GB - A - 2 040 690 (MANUFACTURAS MEDICAS SA)<br>* Text and figures * | 1,6 | A 61 M 17/00 |
| A | US - A - 4 176 666 (T. HOVEY)<br>* Text and figures * | 1,4,6 | |
| A | US - A - 4 180 066 (R. MILLIKEN et al.)<br>* Figures; column 3, line 51 - column 4, line 38 * | 1,4,6 | TECHNICAL FIELDS SEARCHED (Int.Cl. ³) |
| A | US - A - 4 036 221 (D. HILLSMAN et al.)<br>* Figure 1; column 8, lines 16-38; column 9, lines 8-25 * | 1-3 | A 61 M |
| A | US - A - 4 182 599 (T. EYRICK et al.)<br>* Figure 1; column 11, lines 1-33; column 12, lines 59-63 * | 1,2 | |
| A | FR - A - 2 227 021 (TECNA CORP.)<br>* Figures 1,2,6; page 5, lines 9-20; page 10, lines 14-19 * | 1-3 | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 18-03-1982 | VEREECKE |

EPO Form 1503.1 06.78